# EUROPÄISCHE PATENTSCHRIFT

(11) **EP 1 596 749 B1**
(45) Veröffentlichungstag und Bekanntmachung des Hinweises auf die Patenterteilung: **05.11.2008**
(21) Anmeldenummer: 04713895.3
(22) Anmeldetag: 24.02.2004
(51) Int. Cl.: A61B 19/00, A61F 2/46, G01S 5/18

(54) **PATELLA-REFERENZVORRICHTUNG**
PATELLA-REFERENCING DEVICE
DISPOSITIF DE REFERENCIATION DE ROTULE

(30) Priorität: 26.02.2003 DE 10309500
(43) Veröffentlichungstag der Anmeldung: 23.11.2005
(73) Patentinhaber: Aesculap AG, 78532 Tuttlingen (DE)
(72) Erfinder: BÖTTIGER, Roland, 78604 Rietheim-Weilheim (DE); LEITNER, François, F-38410 Uriage (FR)
(74) Vertreter: Pioch, Holger
(86) Internationale Anmeldenummer: PCT/EP2004/001806
(87) Internationale Veröffentlichungsnummer: WO 2004/075767

(56) Entgegenhaltungen:
- WO-A-02/36031
- FR-A- 2 799 634
- US-A- 5 249 581
- US-A- 5 667 512
- US-A- 2002 035 321
- US-A1- 2001 036 245
- US-A1- 2002 068 942

## Beschreibung

Die Erfindung betrifft eine Patella-Referenzvorrichtung zum Bestimmen der räumlichen Lage einer Patella eines menschlichen Kniegelenks bei einem navigationsgestützten chirurgischen Eingriff.

Navigationsgestützte chirurgische Eingriffe werden in vielfältiger Weise am menschlichen Körper durchgeführt. Schwerpunkt derartiger Eingriffe sind Operationen, bei denen Gelenke des menschlichen Körpers ganz oder teilweise durch künstliche Gelenke ersetzt werden. Ein Beispiel hierfür ist eine Operation zum Ersetzen eines teilweise zerstörten menschlichen Kniegelenks durch eine Kniegelenkendoprothese. Bei bekannten Verfahren wird zur Vorbereitung von Sägeschnitten am Kniegelenk ein Referenzelement am Femur und ein weiteres an der Tibia festgelegt. Durch Bestimmen von Gelenkpunkten, insbesondere auch von Gelenkpunkten benachbarter Gelenke des Kniegelenks, nämlich des Hüftgelenks und des Sprunggelenks, können zum Einsetzen der Endoprothese erforderliche Sägeschnitte berechnet und navigationsgestützt durchgeführt werden.

Schwierigkeiten ergeben sich bei derartigen Operationen dann, wenn die Patella nicht vollständig entfernt wird, sondern zusammen mit der Patellasehne erhalten bleibt. Insbesondere kann es vorkommen, daß die Lauffläche der Patella, also deren Rückseite, nicht optimal zum künstlichen Kniegelenk paßt. Sie muß dann entsprechend bearbeitet werden, um eine optimale Patellabewegung zu gewährleisten. Als Anhaltspunkte für eine Bearbeitung der Patellarückseite bleiben einem Operateur hauptsächlich seine Erfahrung und sein Gefühl.

Aus der WO 02/36031 A1 ist es bekannt, eine Referenzvorrichtung an einer Patella anzubringen. Dieses Dokument offenbart die Merkmale des Oberbegriffs des Anspruchs 1. Des Weiteren ist in der US 2001/0036245 A1 eine Referenzvorrichtung offenbart mit einem Grundkörper, einem von einer Nachweisvorrichtung detektierbaren Referenzelement und einer Verbindung zwischen dem Grundkörper und dem Referenzelement. Und schließlich ist in der US 2002/0068942 A1 eine Referenzvorrichtung beschrieben, die in beliebig viele Referenzstellungen verdreht werden kann.

Daher ist es Aufgabe der vorliegenden Erfindung, eine Patella-Referenzvorrichtung der eingangs beschriebenen Art vorzuschlagen, mit weicher navigationsgestützte Knieoperationen vereinfacht und optimiert werden können.

Diese Aufgabe wird mit einer Patella-Referenzvorrichtung der eingangs beschriebenen Art gelöst, umfassend einen an der Patella festlegbaren Grundkörper, ein von einer Nachweisvorrichtung detektierbares Referenzelement und eine Verbindungsvorrichtung zum lösbaren Verbinden des Grundkörpers und des Referenzelements in einer ersten Referenzstellung und in nur einer einzigen weiteren, von der ersten Referenzstellung abweichenden zweiten und gegenüber der ersten Referenzstellung um 180° gedrehten Referenzstellung.

Die Vorrichtung an sich und deren Ausgestaltung haben den Vorteil, daß die Patella-Referenzvorrichtung nach Eröffnen eines Zugangs zu der Patella an dieser fixiert und eine Bewegung derselben während einer Kniebeugung und -streckung referenziert werden kann. Ferner kann die Lage der Patella relativ zu den Referenzpunkten an Femur und Tibia bestimmt werden, aus welchen sich die Beinachse und eine relative Lage zum Gelenkspalt ermitteln lassen. In jedem Fall verbleibt die Patella-Referenzvorrichtung während der gesamten Operation an der Patella, so daß deren Position stets reproduzierbar bleibt. Da die Patellasehne zur Bearbeitung von Femur und Tibia am eröffneten Kniegelenk üblicherweise nach lateral weggeklappt wird, wobei deren Lauffläche dann nach oben zeigt, ist es mit der vorliegenden Erfindung möglich, die Patella auch in ihrer weggeklappten Position zu navigieren, denn das Referenzelement kann in mindestens einer weiteren Referenzstellung mit dem Grundkörper verbunden werden. Ist beispielsweise das Referenzelement zunächst in einem

Bereich der Patellavorderseite positioniert, so kann es zum Beispiel vor einem lateralen Wegklappen der Patellasehne vom Grundkörper gelöst und in einer zweiten Referenzstellung wieder mit diesem verbunden werden. Dies könnte beispielsweise zu einer Anordnung führen, bei welcher sich das Referenzelement dann im wesentlichen in einem Bereich vor der Patellarückseite befindet, also im Bereich von deren Lauffläche. In jedem Fall ist es möglich, und zwar unabhängig von einer Lage der Patella, vor, während oder nach dem chirurgischen Eingriff, diese stets eindeutig zu navigieren. Dies bedeutet, daß eine Patellabewegung verfolgt werden kann und entsprechende Korrekturen an dieser vorgenommen werden können, und zwar so lange, bis eine Bewegung der Patella in Verbindung mit dem künstlichen Kniegelenk einer gewünschten Bahn folgt.

Besonders einfach wird der Aufbau der Referenzvorrichtung, wenn der Grundkörper ein erstes Rahmenelement und mindestens ein zweites Rahmenelemente aufweist, wobei in einer Lösestellung das mindestens eine zweite Rahmenelement relativ zum ersten Rahmenelement bewegbar ist und wobei in einer Fixierstellung das erste Rahmenelement und das mindestens eine zweite Rahmenelement unbeweglich aneinander fixiert sind. Mit dieser Anordnung läßt sich eine besonders einfache Fixierung des Grundkörpers an der Patella erreichen.

Günstig ist es, wenn das erste Rahmenelement und/oder das mindestens eine zweite Rahmenelement eine Führung für das jeweils andere Rahmenelement umfassen zum Führen einer Relativbewegung des ersten und des mindestens einen zweiten Rahmenelements in der Lösestellung. Damit läßt sich ein unbeabsichtigtes Lösen der mindestens zwei Rahmenelemente voneinander verhindern. Außerdem ist durch die vorgesehene Führung eine Einhandbedienung der Referenzvorrichtung möglich.

Vorteilhaft ist es, wenn das erste Rahmenelement relativ zum mindestens einem zweiten Rahmenelement verschiebbar ist. Dadurch läßt sich insgesamt eine besonders kleine Baugröße der Referenzvorrichtung erreichen. Ferner ist die Referenzvorrichtung dadurch besonders einfach zu bedienen.

Denkbar wäre es, daß die Führung eine geradlinige Bahn definiert. Besonders günstig ist es jedoch, wenn die Führung eine gekrümmte Bahn für eine Relativbewegung des ersten und des mindestens einen zweiten Rahmenelemente in der Lösestellung definiert. Dies ist insbesondere dann von Vorteil und ermöglicht eine besonders kleine Baugröße der Referenzvorrichtung, wenn die mindestens zwei Rahmenelemente gebogen sind. Gebogene Rahmenelemente passen sich besonders gut an eine gewölbte Form der Patella an und ermöglichen einen besonders kompakten Aufbau der Referenzvorrichtung.

Um ein einfaches Überführen der Referenzvorrichtung von der Lösestellung in die Fixierstellung zu erreichen, kann eine Rastvorrichtung vorgesehen sein zum lösbaren Verbinden des ersten Rahmenelements mit dem mindestens einen zweiten Rahmenelement in der Fixierstellung. Die mindestens zwei Rahmenelemente werden dadurch in ihrer Stellung relativ zueinander fixiert, beispielsweise indem ein Rastvorsprung in eine Rastausnehmung eingreift. Bei einer Vielzahl von Rastausnehmungen können dann eine Vielzahl von fixierstellungen eingenommen werden, so daß die Referenzvorrichtung an Patellen unterschiedlicher Größe festgelegt werden kann.

Besonders einfach läßt sich die Referenzvorrichtung bedienen, wenn die Rastvorrichtung ein Betätigungselement umfaßt zum Überführen der Rastvorrichtung von der Lösestellung in die Fixierstellung und umgekehrt. Allein durch Betätigen des Betätigungselements kann damit eine Verbindung zwischen den mindestens zwei Rahmenelementen gelöst und wieder fixiert werden.

Damit die Referenzvorrichtung sich nicht unabsichtlich von der Patella lösen kann, ist es vorteilhaft, wenn die Rastvorrichtung in einer Grundstellung die Fixierstellung einnimmt.

Gemäß einer bevorzugten Ausführungsform der Erfindung kann vorgesehen sein, daß die Rastvorrichtung ein vorspannendes Element umfaßt zum automatischen Überführen des Betätigungselements in die Grundstellung. Ist die Referenzvorrichtung an der Patella festgelegt, so bewirkt das vorspannende Element, beispielsweise eine Feder oder ein beliebiges anderes elastisches Element, daß sich die Referenzvorrichtung nicht von der Patella lösen kann.

Um eine eindeutige Fixierung der Referenzvorrichtung an der Patella zu gewährleisten, ist es günstig, wenn am Grundkörper mindestens drei Anlagepunkte zum Anlegen an die Patella vorgesehen sind, wobei die mindestens drei Anlagepunkte eine Basisebene definieren. Eine Ebene wird besonders einfach und eindeutig dann definiert, wenn drei Anlagepunkte vorgesehen sind. Ferner ergibt sich dadurch auch eine optimale Fixierung der Referenzvorrichtung an der Patella.

Ein besonders guter Halt der Referenzvorrichtung an der Patella ergibt sich, wenn mindestens einer der Anlagepunkte durch eine Spitze eines Dorns definiert ist. Dieser kann sich etwas in die Patella eingraben und wird dort sicher gehalten.

Besonders sicher wird die Referenzvorrichtung an der Patella fixiert, wenn alle Anlagepunkte durch eine Spitze eines Dorns definiert sind.

Um einen optimalen Halt der Referenzvorrichtung an der Patella zu erreichen, tragen das erste Rahmenelement und das mindestens zweite Rahmenelement jeweils mindestens einen Dorn. Dies ermöglicht es, daß die Patella zwischen den Dornen der mindestens zwei Rahmenelemente gehalten wird. Dabei ist es günstig, wenn die Dorne so an den mindestens zwei Rahmenelementen angeordnet sind, daß die Rahmenelemente zusammen mit den Dornen die Patella mindestens teilweise umgreifen, insbesondere ist dies möglich, wenn in einer Seitenansicht der Grundkörper im wesentlichen ein U-förmiges Profil aufweist.

Um den Grundkörper besonders sicher an der Patella festzulegen, kann mindestens ein Dorn relativ zu einem Rahmenelement verstellbar angeordnet sein. Beispielsweise könnte ein Dorn relativ zu einem der Rahmenelemente verdrehbar angeordnet sein.

Besonders sicher wird die Patella an der Referenzvorrichtung gehalten, wenn die Anlagepunkte im wesentlichen aufeinander zu weisen. Besonders dann, wenn die Anlagepunkte durch Spitzen von Dornen definiert werden, weisen die Dornspitzen im wesentlichen aufeinander zu und halten die Patella zwischen sich.

Vorteilhaft ist es, wenn das Referenzelement mindestens drei von der Nachweisvorrichtung detektierbare Sendeelemente trägt. Damit läßt sich eine Position des Referenzelements im Raum eindeutig bestimmen.

Grundsätzlich könnten die Sendeelemente aktive Sendeelemente sein. Günstig ist es jedoch, wenn die mindestens drei Sendeelemente passiver Art sind. Dadurch kann auf eine Energieversorgung der Sendeelemente verzichtet werden.

Besonders einfach lassen sich die Sendeelemente nachweisen, wenn diese elektromagnetische Strahlung reflektierende Kugeln sind.

Vorzugsweise sind die mindestens drei Sendeelemente so angeordnet, daß sie eine Referenzelementebene definieren. Damit läßt sich eine Bahnbewegung der Patella besonders einfach navigationsgestützt verfolgen.

Gemäß einer bevorzugten Ausführungsform der Erfindung kann vorgesehen sein, daß die Referenzelementebene in der ersten Referenzstellung parallel zur Referenzelementebene in der mindestens einen zweiten Referenzstellung verläuft. Dies bedeutet, daß das Referenzelement in der ersten Referenzstellung und in der zweiten Referenzstellung so mit dem Grundköper verbindbar ist, daß die Referenzelementebene stets parallel zu sich selbst verläuft. Sie kann beispielsweise auch in beiden Stellungen parallel zur Basisebene verlaufen. Damit läßt sich die Patella in einer lateral weggeklappten Stellung genau so detektieren wie in einer natürlichen Position der Patella am Knie.

Vorzugsweise weist das Referenzelement einen Referenzelementgrundkörper auf und sind die mindestens drei Sendeelemente am Referenzelementgrundkörper gehalten. Diese Ausgestaltung ermöglicht es, den Referenzelementgrundkörper so auszuformen, daß er möglichst platzsparend am Grundkörper angeordnet werden kann.

Günstig ist es, wenn die mindestens drei Sendeelemente am Referenzelementgrundkörper lösbar gehalten sind. Sie können dann bei Bedarf abgenommen und durch andere Sendeelemente ausgetauscht werden.

Ein besonders einfacher Aufbau des Referenzelements ergibt sich, wenn der Referenzelementgrundkörper die Form eines ebenen, flachen Rings aufweist. Ein solcher Ring ist einfach herzustellen und gestattet insgesamt eine besonders kleine Baugröße der Referenzvorrichtung. Zudem wird ein Operationsbereich nur minimal abgedeckt.

Damit der Grundkörper und das Referenzelement besonders einfach und sicher verbunden werden können, kann vorgesehen sein, daß die Verbindungsvorrichtung ein erstes Kupplungselement und ein zweites Kupplungselement umfaßt, wobei das erste Kupplungselement am Referenzelement und das zweite Kupplungselement am Grundkörper angeordnet ist.

Um eine eindeutige relative Stellung zwischen den mindestens zwei Rahmenelementen zu gewährleisten, ist es günstig, wenn das erste Kupplungselement und das zweite Kupplungselement formschlüssig ineinander greifen, wenn die Referenzvorrichtung die erste und/oder die mindestens eine zweite Referenzstellung einnimmt. Ist die Form und die Lage der Patella relativ zum Referenzelement einmal bestimmt, kann auch beim Überführen des Referenzelements relativ zum Grundkörper von der ersten Referenzstellung in die zweite Referenzstellung und umgekehrt stets die Position und die Form der Patella navigiert werden.

Ein besonders einfacher Aufbau der Referenzvorrichtung ergibt sich, wenn das erste Kupplungselement am Referenzelementgrundkörper und das zweite Kupplungselement am ersten Rahmenelement angeordnet ist.

Gemäß einer bevorzugten Ausführungsform der Erfindung kann vorgesehen sein, daß das erste Kupplungselement relativ zum zweiten Kupplungselement in einer Kupplungsrichtung bewegbar ist zum Überführen des ersten und des zweiten Kupplungselement von einer Entkupplungsstellung, in welcher das erste und das zweite Kupplungselement voneinander getrennt sind, in eine Kupplungsstellung, in welcher die Referenzvorrichtung die erste und/oder die mindestens eine zweite Referenzstellung einnimmt. Dadurch lassen sich eindeutig Kupplungspositionen und gewünschte Referenzstellungen definieren.

Besonders einfach wird das Zusammenfügen des Grundkörpers und des Referenzelements, wenn die Kupplungsrichtung parallel zur Basisebene und/oder parallel zur Referenzelementebene verläuft. Dies gestattet es, wenn Basisebene beispielsweise im wesentlichen parallel zur einer von der Patella definierten Patellaebene verläuft, zunächst den Grundkörper an der Patella zu fixieren und danach das Referenzelement in der Kupplungsrichtung an den Grundkörper heranzuführen, zum Beispiel von lateral oder medial kommend. Denkbar wäre es auch, das Referenzelement von der ersten Referenzstellung in die zweite Referenzstellung zu überführen, ohne das Referenzelement vom Grundkörper zu lösen, beispielsweise durch Verschwenken um eine Schwenkachse, welche parallel zur Kupplungsrichtung verlaufen kann.

Vorteilhaft ist es, wenn das erste Kupplungselement eine Ausnehmung und das zweite Kupplungselement einen in die Ausnehmung einführbaren Vorsprung umfaßt. Dies ergibt einen besonders einfachen Aufbau der beiden Kupplungselemente und damit der gesamten Referenzvorrichtung. Außerdem kann durch die Form der Ausnehmung bzw. des Vorsprungs die Zahl der Referenzstellungen vorgegeben werden.

Um ein unbeabsichtigtes Lösen des Referenzelements vom Grundkörper zu verhindern, kann ein Verriegelungselement vorgesehen sein zum Verriegeln des ersten und des zweiten Kupplungselements in der Kupplungsstellung.

Eine besonders einfache Bedienung sowie ein einfacher Aufbau der Referenzvorrichtung ergibt sich, wenn der Vorsprung mit einem Gewinde versehen und das Verriegelungselement eine Mutter mit einem zum Gewinde korrespondierenden Innengewinde ist.

Die nachfolgende Beschreibung einer bevorzugten Ausführungsform der Erfindung dient im Zusammenhang mit der Zeichnung der näheren Erläuterung. Es zeigen:
- Figur 1:: eine schematische Darstellung einer navigierten Knieoperation an einem Patient;
- Figur 2:: eine Ansicht eines eröffneten Kniegelenks von vorn mit Referenz- elementen an Tibia, Femur und Patella;
- Figur 3:: eine perspektivische Ansicht einer Patella-Referenzvorrichtung;
- Figur 4:: eine Seitenansicht eines Grundkörpers der Patella- Referenzvorrichtung; und
- Figur 5:: eine Ansicht des Grundkörpers aus Figur 4 in Richtung des Pfeils A.

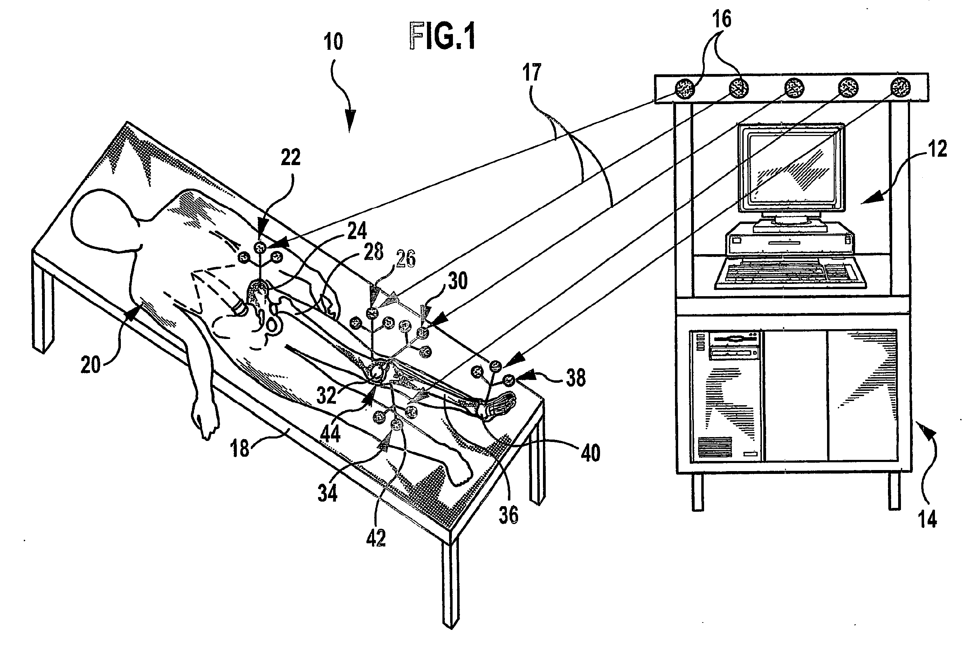

In Figur 1 ist ein insgesamt mit dem Bezugszeichen 10 versehenes Navigationssystem zum Durchführen navigierter chirurgischer Eingriffe am menschlichen Körper dargestellt. Es umfaßt eine von einem Rechner gesteuerte Sende- und Empfangsstation 14, welche mehrere Sende-/Empfangseinheiten 16 aufweist zum Aussenden und Empfangen von elektromagnetischer Strahlung, sowie mehrere Referenzelemente zum Fixieren an einem Patienten.

Zum Durchführen einer navigierten Knieoperation sind an einem auf einem Operationstisch 18 ruhenden Patienten 20 fünf Referenzelemente fixiert, und zwar ein Hüftmarker 22 am Beckenknochen 24, ein Femurmarker 26 im knienahen Bereich eines Femurs 28, ein erfindungsgemäßer Patellamarker 30 an der Patella 32, ein Tibiamarker 34 im knienahen Bereich einer Tibia 36 und ein Sprunggelenkmarker 38 an einem Sprunggelenk 40. Jeder der genannten Marker 22, 26, 30, 34, 38 umfaßt drei identische Reflektorkugeln 42, welche von den Sende-/Empfangseinheiten 16 ausgesendete elektromagnetische

Strahlung 17 reflektieren, die wiederum von den Sende-/Empfangseinheiten 16 empfangen werden kann.

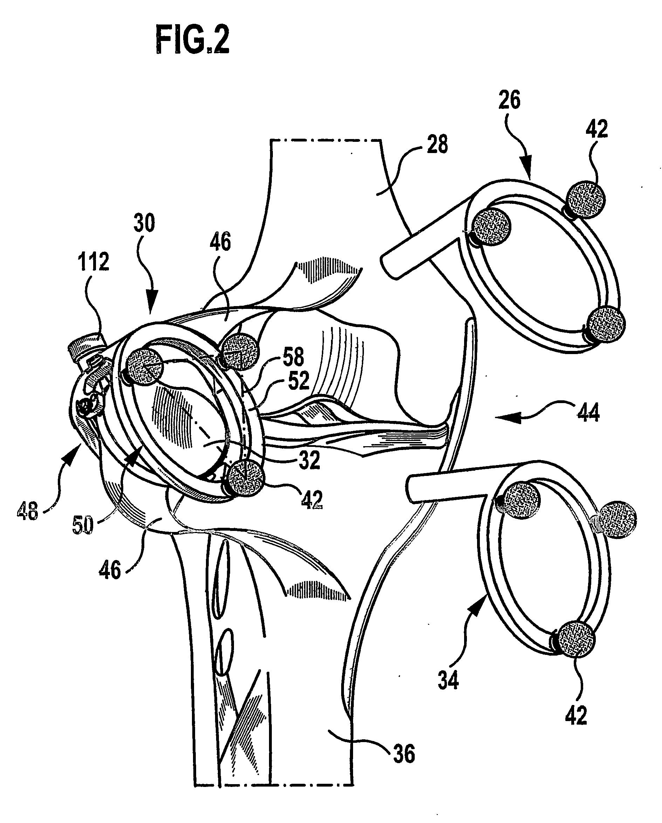

In figur 2 ist ein eröffnetes Kniegelenk 44 dargestellt mit dem im knienahen Bereich des Femur 28 angeordneten Femurmarker 26, mit dem im knienahen Bereich der Tibia 36 angeordneten Tibiamarker 34 und mit dem an der Patella angeordneten Patellamarker 30. Zur Bearbeitung des Knies ist, wie in Figur 2 dargestellt, die Patella 32 nach lateral weggeklappt, so daß eine die Patella 32 tragende und einerseits im knienahen Bereich des Femurs 28 und andererseits im knienahen Bereich der Tibia 36 angewachsene Patellasehne 46 etwas verdrillt wird.

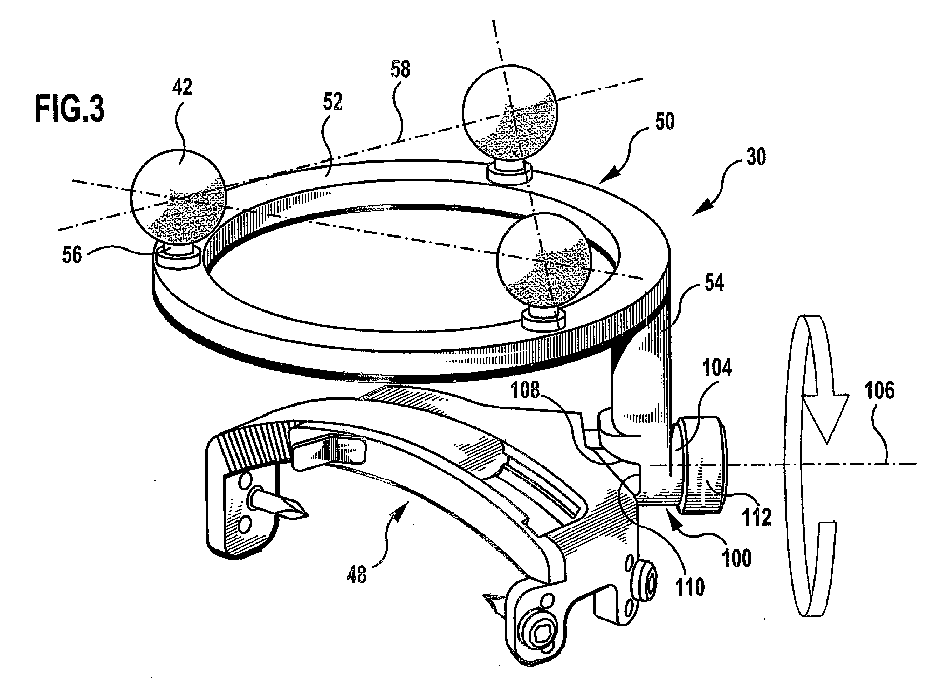

In Figur 2 nimmt der Patellamarker 30 eine zweite Referenzstellung ein, in Figur 3 eine erste Referenzstellung, welche weiter unten näher definiert werden.

Ein Aufbau des Patellamarkers 30 wird nachfolgend anhand der Figuren 3 bis 5 näher erläutert.

Der Patellamarker 30 umfaßt als wesentliche Grundelemente eine Klammer 48 und einen Reflektoraufsatz 50. Der Reflektoraufsatz 50 ist im wesentlichen in Form eines flachen ebenen Rings 52 ausgebildet, von dem sich senkrecht zu einer vom Ring definierten Ebene ein zylindrischer Abstandsbolzen 54 weg erstreckt. In entgegengesetzter Richtung zum Abstandsbolzen 54 sind gleichmäßig verteilt am Ring 52 drei Halterungen 56 senkrecht abstehend angeordnet, auf welche jeweils eine Reflektorkugel 42 aufgesteckt ist. Jede Reflektorkugel 42 wird an einer Halterung 56 entweder durch einen Klemmsitz oder durch eine lösbare Schnappverbindung gehalten. Mittelpunkte der Reflektorkugeln 42 definieren eine Referenzelementebene 58, welche beim vorliegenden Ausführungsbeispiel parallel zu der vom Ring 52 aufgespannten Ebene verläuft.

Die Klammer 48 ist im wesentlichen zweiteilig aufgebaut. Sie umfaßt einen im wesentlichen U-förmigen Klammerkörper 60 mit zwei parallel zueinander verlaufenden, kreisbogenförmig gekrümmten Schenkeln 62 und 64 sowie einer die beiden Schenkel 62 und 64 verbindenden Platte 66. Der Schenkel 62 weist in einer auf den Schenkel 64 hin weisenden ebenen Seitenfläche 68 eine schwalbenschwanzförmige, kreisbogenförmig gekrümmte Nut 70 auf. In dieser wird ein korrespondierender, schwalbenschwanzförmiger Vorsprung 72 eines zwischen den beiden Schenkeln 62 und 64 geführten Klemmbackens 74 geführt. Der Klemmbacken 74 kann im wesentlichen vollständig zwischen die beiden Schenkel 62 und 64 eingeführt werden und füllt dann das freie, von diesen definierte Volumen vollständig aus.

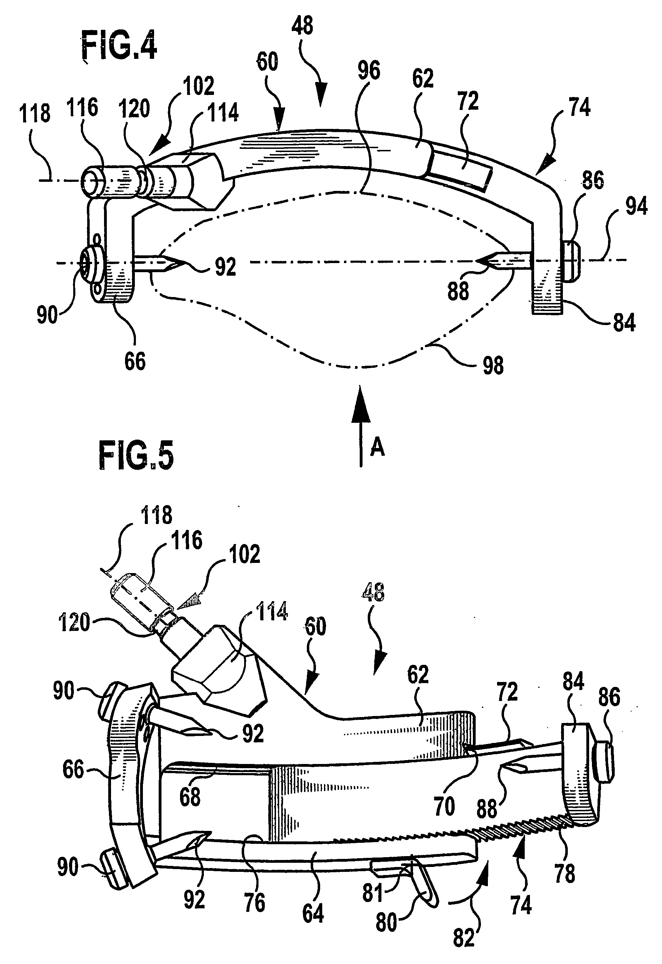

Eine Seitenfläche 76 des Schenkels 64, welche auf die Seitenfläche 68 des Schenkels 62 hin weist, ist im wesentlichen glatt ausgebildet. An ihr liegt eine quer verzahnte Seitenfläche 78 des Klemmbackens 74 an. Zur Fixierung einer Relativstellung zwischen dem Klemmbacken 74 und dem Klammerkörper 60 ist ein am Schenkel 64 um eine Drehachse 81 schwenkbar gelagerter Lösehebel 80 angeordnet, welcher eine nicht dargestellte, zur verzahnten Seitenfläche 78 korrespondierende Verzahnung trägt, die in der in Figur 5 dargestellten Position in die Verzahnung der Seitenfläche 78 eintauchen kann. Wird der Lösehebel 80 in Richtung des in Figur 5 dargestellten Pfeils 82 verschwenkt, so wird die formschlüssige Verbindung zwischen der Verzahnung des Lösehebels und der Verzahnung der Seitenfläche 78 gelöst und der Klemmbacken 74 kann relativ zum Klammerkörper 60 verschoben werden. Die Klammer 48 nimmt dann die Lösestellung ein. Wird der Lösehebel 80 losgelassen, so nimmt er automatisch aufgrund eines nicht dargestellten, mit dem Lösehebel 80 zusammenwirkenden vorspannenden Elements wieder die Fixierstellung ein, das heißt die Verzahnungen des Lösehebels 80 und der Seitenfläche 78 greifen wieder ineinander.

Um die Klammer 48 an der Patella 32 zu befestigen, ist an einem freien Ende des Klemmbackens 74 eine Halteplatte 84 quer abstehend angeordnet. In dieser ist ein mit einem Schraubengewinde versehener Haltedorn 86 gehalten, dessen Spitze 88 einen Anlagepunkt an der Patella 32 definiert und dessen Spitze 88 in Richtung auf die Platte 66 hin weist. In ähnlicher Weise sind an der Platte 66 zwei Haltedorne 92 angeordnet, deren Längsachsen relativ zueinander geneigt sind und deren Spitzen 92 in etwa auf die Spitze 88 des Haltedorns 86 hin weisen. Die drei Spitzen 88 und 92 der Haltedorne 86 und 90 definieren eine Basisebene 94. Bei zusammengefügter Klammer 48, das heißt wenn der Klammerkörper 60 und der Klemmbacken 74 zusammengefügt sind, sind die Platte 66 und die Halteplatte 84 im wesentlichen parallel zueinander ausgerichtet. Die Haltedorne 86 und 90 können relativ zur Halteplatte 84 bzw. zur Platte 66 justiert werden durch Verdrehen der Dorne relativ zu den Platten, was durch korrespondierende Gewinde ermöglicht wird.

Wie in Figur 4 dargestellt, ergibt sich somit in einer Seitenansicht eine im wesentlichen U-förmige Ausgestaltung der Klammer 48, wobei die Platte 66 und die Halteplatte 84 im wesentlichen parallel zueinander angeordnete Schenkel bilden. Die Krümmungen der Schenkel 62 und 64 sowie des Klemmbacken 74 sind so gewählt, daß die Klammer 48 möglichst dicht an eine gekrümmte Vorderseite 96 der Patella 32 angepaßt ist. Eine Rückseite 98 der Patella 32, welche gleichzeitig eine Lauffläche der Patella 32 bildet, ist nach Fixierung der Klammer 48 der Patella frei zugänglich und kann in gewünschter Weise bearbeitet werden.

Zur Verbindung der Klammer 48 und des Reflektoraufsatzes 50 sind diese jeweils mit einem Kupplungselement 100 beziehungsweise 102 versehen. Das Kupplungselement 100 ist an einem freien Ende des Abstandsbolzens 54 angeordnet und umfaßt eine quer zu einer Längsachse des Abstandsbolzens 54 mit einer Bohrungen versehene Hülse 104, wobei die Bohrung eine Symmetrieachse 106 definiert. Ein Ende der Hülse 104 ist zylindrisch ausgebildet, wohingegen das andere Ende der Hülse eine sich V-förmig erweiternde Nut 108 trägt, welche eine Nutboden 110 umfaßt, der senkrecht zur Symmetrieachse 106 verlaufend angeordnet ist. Über dem zylindrischen Ende der Hülse 104 ist ein Knopf 112 federn vorgespannt in Richtung auf die Nut 108 hin gehalten. Er kann etwas vom Abstandsbolzen 54 weg zurückgezogen werden, so daß nicht näher dargestellte Rastelemente quer zur Symmetrieachse 106 von dieser zurückgezogen werden.

Das Kupplungselement 102 ist seitlich abstehend am Schenkel 62 angeordnet und umfaßt einen zur Nut 108 korrespondierenden Klemmkörper 114, welcher formschlüssig in die Nut 108 eintauchen kann. Der Klemmkörper 114 trägt einen Haltestift 116, welcher eine parallel zur Basisebene 94 verlaufende Symmetrieachse 118 aufweist. Am Haltestift 116 ist eine Ringnut 120 angeordnet.

Zum Zusammenfügen der Klammer 48 und des Reflektoraufsatzes 50 wird der Haltestift 116 durch die Bohrung der Hülse 104 gesteckt, bis die nicht dargestellten Rastelemente am Kupplungselement 100 in die Ringnut 102 eintauchen. Der Klemmkörper 114 taucht dann auch formschlüssig in die Nut 108 ein, so daß die Symmetrieachsen 106 und 118 zusammenfallen. Durch die Ausgestaltung des Kupplungselements 100, insbesondere durch die Vorsehung der Nut 108, können die Klammer 48 und der Reflektoraufsatz 50 in zwei unterschiedlichen Stellungen, nämlich einer ersten Referenzstellung, wie sie in Figur 3 dargestellt ist, und einer zweiten Referenzstellung, wie sie in Figur 2 dargestellt ist, miteinander verbunden werden. Zum Überführen des Patellamarkers 30 von der ersten Referenzstellung in die zweite Referenzstellung wird der Knopf 112 zurückgezogen, bis die Rastelemente die Ringnut 120 freigeben und eine Rotation des Reflektoraufsatzes 50 um die Symmetrieachsen 106 beziehungsweise 118 möglich ist. Nach einer Drehung um 180° kann der Klemmkörper 114 wieder in die Nut 110 eintauchen und die Rastelemente durch Eintauchen in die Ringnut 120 den Reflektoraufsatz 50 an der Klammer 48 in der zweiten Referenzstellung fixieren.

In der zweiten Referenzstellung, wie sie in Figur 2 dargestellt ist, umgibt der Ring 52 die Rückseite 98 der Patella 32. Eine Bearbeitung der Rückseite 98 ist dennoch möglich, da der Ring 52 im wesentlichen einen freien Zugang zur Rückseite 98 ermöglicht.

Zur Aufnahme und zum Verfolgen einer ursprünglichen natürlichen Bewegung der Patella 32 wird der Patellamarker in der in Figur 3 dargestellten ersten Referenzstellung an der Patella 32 befestigt, und zwar durch eine relative Verschiebung des Klammerkörpers 60 und des Klemmbackens 74 bis die Spitzen 88 und 92 der Haltedorne 86 und 90 so weit in die Patella 32 eindringen, daß die Klammer 48 fest mit der Patella 32 verbunden ist. Zur Bearbeitung von Femur 28 und Tibia 36 wird die Patella 32, wie in Figur 2 dargestellt, lateral weggeklappt. Um die Position der Patella 32 auch in der weggeklappten Stellung nachweisen zu können, wird der Reflektoraufsatz 50 relativ zur Klammer 48 um 180° verschwenkt, so daß er die in Figur 2 dargestellte zweite Referenzstellung einnimmt. Nach Bearbeitung der Patella 32 und Austausch von Teilen des Kniegelenks 42 wird der Patellamarker 30 wieder in die erste Referenzstellung gebracht und die Patella 32 in ihre natürliche Stellung zurückgeklappt. Eine Bewegung der Patella 32 relativ zu eingesetzten Kniegelenkteilen kann während einer Beuge- und Streckbewegung des Kniegelenks 44 aufgenommen werden.

## Patentansprüche

1. Patella-Referenzvorrichtung (30) zum Bestimmen der räumlichen Lage einer Patella (32) eines menschlichen Kniegelenks (44) bei einem navigationsgestützten chirurgischen Eingriff, umfassend einen an der Patella (32) festlegbaren Grundkörper (48), ein von einer Nachweisvorrichtung (14) detektierbares Referenzelement (50) **dadurch gekennzeichnet dass**, die Patella-Referenzvorrichtung (30) weiter eine Verbindungsvorrichtung (100, 102) zum lösbaren Verbinden des Grundkörpers (48) und des Referenzelements (50) in einer ersten Referenzstellung und in nur einer einzigen weiteren, von der ersten Referenzstellung abweichenden zweiten und gegenüber der ersten um 180° gedrehten Referenzstellung, umfasst.

2. Referenzvorrichtung nach Anspruch 1, wobei der Grundkörper (48) ein erstes Rahmenelement (60) und mindestens ein zweites Rahmenelement (74) aufweist, wobei in einer Lösestellung das mindestens eine zweite Rahmenelement (74) relativ zum ersten Rahmenelement (60) bewegbar ist und wobei in einer Fixierstellung das erste Rahmenelement (60) und das mindestens eine zweite Rahmenelement (74) unbeweglich aneinander fixiert sind.

3. Referenzvorrichtung nach Anspruch 2, wobei das erste Rahmenelemente (60) und/oder das mindestens eine zweite Rahmenelement (74) eine Führung (70, 72) für das jeweils andere Rahmenelement (60, 74) umfassen zum Führen einer Relativbewegung des ersten und des mindestens einen zweiten Rahmenelements (60, 74) in der Lösestellung.

4. Referenzvorrichtung nach einem der Ansprüche 2 oder 3, wobei das erste Rahmenelement (60) relativ zum mindestens einen zweiten Rahmenelement (74) verschiebbar ist.

5. Referenzvorrichtung nach einem der Ansprüche 3 oder 4, wobei die Führung (70, 72) eine gekrümmte Bahn für eine Relativbewegung des ersten und des mindestens einen zweiten Rahmenelements (60, 74) in der Lösestellung definiert.

6. Referenzvorrichtung nach einem der Ansprüche 2 bis 5, wobei eine Rastvorrichtung (80) vorgesehen ist zum lösbaren Verbinden des ersten Rahmenelements (60) mit dem mindestens einen zweiten Rahmenelement (74) in der Fixierstellung.

7. Referenzvorrichtung nach Anspruch 6, wobei die Rastvorrichtung ein Betätigungselement (80) umfaßt zum Überführen der Rastvorrichtung von der Lösestellung in die Fixierstellung und umgekehrt.

8. Referenzvorrichtung nach Anspruch 7, wobei die Rastvorrichtung (80) in einer Grundstellung die Fixierstellung einnimmt.

9. Referenzvorrichtung nach Anspruch 8, wobei die Rastvorrichtung ein vorspannendes Element umfaßt zum automatischen Überführen des Betätigungselements (80) in die Grundstellung.

10. Referenzvorrichtung nach einem der voranstehenden Ansprüche, wobei am Grundkörper (48) mindestens drei Anlagepunkte (88, 92) zum Anlegen an die Patella (32) vorgesehen sind, wobei die mindestens drei Anlagepunkte (88, 92) eine Basisebene (94) definieren.

11. Referenzvorrichtung nach Anspruch 10, wobei mindestens einer der Anlagepunkte (88, 92) durch eine Spitze (88, 92) eines Dorns (86, 90) definiert ist.

12. Referenzvorrichtung nach Anspruch 11, wobei alle Anlagepunkte (88, 92) durch eine Spitze (88, 92) eines Dorns (86, 90) definiert sind.

13. Referenzvorrichtung nach einem der Ansprüche 11 oder 12, wobei das erste Rahmenelement (60) und das mindestens zweite Rahmenelement (74) jeweils mindestens einen Dorn (86, 90) tragen.

14. Referenzvorrichtung nach einem der Ansprüche 11 bis 13, wobei mindestens ein Dorn (86, 90) relativ zu einem Rahmenelement (60, 74) verstellbar angeordnet ist.

15. Referenzvorrichtung nach einem der Ansprüche 10 bis 14, wobei die Anlagepunkte (88, 92) im wesentlichen aufeinander zu weisen.

16. Referenzvorrichtung nach einem der voranstehenden Ansprüche, wobei das Referenzelement (50) mindestens drei von der Nachweisvorrichtung (14) detektierbare Sendeelemente (42) trägt.

17. Referenzvorrichtung nach Anspruch 16, wobei die mindestens drei Sendeelemente (42) passive Sendeelemente (42) sind.

18. Referenzvorrichtung nach einem der Ansprüche 16 oder 17, wobei die Sendeelemente elektromagnetische Strahlung reflektierende Kugeln (42) sind.

19. Referenzvorrichtung nach einem der Ansprüche 16 bis 18, wobei die mindestens drei Sendeelemente (42) eine Referenzelementebene (58) definieren.

20. Referenzvorrichtung nach Anspruch 19, wobei die Referenzelementebene (58) in der ersten Referenzstellung parallel zur Referenzelementebene (58) in der mindestens einen zweiten Referenzstellung verläuft.

21. Referenzvorrichtung nach einem der Ansprüche 16 bis 20, wobei das Referenzelement (50) einen Referenzelementgrundkörper (52) aufweist und die mindestens drei Sendeelemente (42) am Referenzelementgrundkörper (52) gehalten sind.

22. Referenzvorrichtung nach Anspruch 21, wobei die mindestens drei Sendeelemente (42) am Referenzelementgrundkörper (52) lösbar gehalten sind.

23. Referenzvorrichtung nach einem der Ansprüche 21 oder 22, wobei der Referenzelementgrundkörper die form eines ebenen, flachen Rings (52) aufweist.

24. Referenzvorrichtung nach einem der voranstehenden Ansprüche, wobei die Verbindungsvorrichtung (100, 102) ein erstes Kupplungselement (100) und ein zweites Kupplungselement (102) umfaßt, wobei das erste Kupplungselement (100) am Referenzelement (50) und das zweite Kupplungselement (102) am Grundkörper (48) angeordnet ist.

25. Referenzvorrichtung nach Anspruch 24, wobei das erste Kupplungselement (100) und das zweite Kupplungselement (102) formschlüssig ineinandergreifen, wenn die Referenzvorrichtung (50) die erste und/oder die mindestens eine zweite Referenzstellung einnimmt.

26. Referenzvorrichtung nach Anspruch 24 oder 25, wobei das erste Kupplungselement (100) am Referenzelementgrundkörper (52) und das zweite Kupplungselement (102) am ersten Rahmenelement (60) angeordnet ist.

27. Referenzvorrichtung nach einem der Ansprüche 24 bis 26, wobei das erste Kupplungselement (100) relativ zum zweiten Kupplungselement (102) in einer Kupplungsrichtung (118) bewegbar ist zum Überführen des ersten und des zweiten Kupplungselements (100, 102) von einer Entkupplungsstellung, in welcher das erste und das zweite Kupplungselement (100, 102) voneinander getrennt sind, in eine Kupplungsstellung, in welcher die Referenzvorrichtung (30) die erste und/oder die mindestens eine zweite Referenzstellung einnimmt.

28. Referenzvorrichtung nach Anspruch 27, wobei die Kupplungsrichtung (118) parallel zur Basisebene (94) und/oder parallel zur Referenzelementebene (58) verläuft.

29. Referenzvorrichtung nach einem der Ansprüche 24 bis 28, wobei das erste Kupplungselement (100) eine Ausnehmung (108) und das zweite Kupplungselement (102) einen in die Ausnehmung (108) einführbaren Vorsprung (114) umfaßt.

30. Referenzvorrichtung nach einem der Ansprüche 24 bis 29, wobei ein Verriegelungselement (112) vorgesehen ist zum Verriegeln des ersten und des zweiten Kupplungselements (100, 102) in der Kupplungsstellung.

31. Referenzvorrichtung nach den Ansprüchen 29 und 30, wobei der Vorsprung mit einem Gewinde versehen und das Verriegelungselement (112) eine Mutter mit einem zum Gewinde korrespondierenden Innengewinde ist.

## Claims

1. Patella reference device (30) for the determination of the spatial position of a patella (32) of a human knee joint (44) during a navigation-assisted surgical procedure, comprising a base unit (48) that can be secured on the patella (32), a reference element (50) that can be detected by a detection device (14), **characterized in that** the patella reference device (30) further comprises a connecting device (100, 102) for the detachable connection of the base unit (48) and the reference element (50) in a first reference position and in only a single further second reference position differing from the first reference position and rotated through 180° in relation to the first one.

2. Reference device according to Claim 1, wherein the base unit (48) has a first frame element (60) and at least one second frame element (74), wherein in a release position the at least one second frame element (74) is movable relative to the first frame element (60), and wherein in a securing position the first frame element (60) and the at least one second frame element (74) are immovably secured on one another.

3. Reference device according to Claim 2, wherein the first frame element (60) and/or the at least one second frame element (74) comprise a guide means (70, 72) for the respective other frame element (60, 74) for guidance of a relative movement of the first and the at least one second frame element (60, 74) in the release position.

4. Reference device according to Claim 2 or 3, wherein the first frame element (60) is displaceable relative to the at least one second frame element (74).

5. Reference device according to Claim 3 or 4, wherein the guide means (70, 72) defines a curved path for a relative movement of the first and the at least one second frame element (60, 74) in the release position.

6. Reference device according to one of Claims 2 to 5, wherein a locking device (80) is provided for the detachable connection of the first frame element (60) to the at least one second frame element (74) in the securing position.

7. Reference device according to Claim 6, wherein the locking device comprises an operating element (80) for moving the locking device from the release position into the securing position and vice versa.

8. Reference device according to Claim 7, wherein the locking device (80) assumes the securing position in a normal position.

9. Reference device according to Claim 8, wherein the locking device comprises a biasing element for automatically moving the operating element (80) into the normal position.

10. Reference device according to one of the preceding claims, wherein at least three application points (88, 92) are provided on the base unit (48) for placement on the patella (32), and the at least three application points (88, 92) define a base plane (94).

11. Reference device according to Claim 10, wherein at least one of the application points (88, 92) is defined by a point (88, 92) of a spike (86, 90).

12. Reference device according to Claim 11, wherein all the application points (88, 92) are defined by a point (88, 92) of a spike (86, 90).

13. Reference device according to Claim 11 or 12, wherein the first frame element (60) and the at least second frame element (74) respectively bear at least one spike (86, 90).

14. Reference device according to one of Claims 11 to 13, wherein at least one spike (86, 90) is adjustably arranged relative to a frame element (60,74).

15. Reference device according to one of Claims 10 to 14, wherein the application points (88, 92) essentially point towards one another.

16. Reference device according to one of the preceding claims, wherein the reference element (50) bears at least three transmission elements (42) detectable by the detection device (14).

17. Reference device according to Claim 16, wherein the at least three transmission elements (42) are passive transmission elements (42).

18. Reference device according to Claim 16 or 17, wherein the transmission elements are spheres (42) reflecting electromagnetic radiation.

19. Reference device according to one of Claims 16 to 18, wherein the at least three transmission elements (42) define a reference element plane (58).

20. Reference device according to Claim 19, wherein in the first reference position the reference element plane (58) runs parallel to the reference element plane (58) in the at least one second reference position.

21. Reference device according to one of Claims 16 to 20, wherein the reference element (50) has a reference element base unit (52) and the at least three transmission elements (42) are held on the reference element base unit (52).

22. Reference device according to Claim 21, wherein the at least three transmission elements (42) are detachably held on the reference element base unit (52).

23. Reference device according to Claim 21 or 22, wherein the reference element base unit has the form of a plane, flat ring (52).

24. Reference device according to one of the preceding claims, wherein the connecting device (100, 102) comprises a first coupling element (100) and a second coupling element (102), and the first coupling element (100) is arranged on the reference element (50) and the second coupling element (102) is arranged on the base unit (48).

25. Reference device according to Claim 24, wherein the first coupling element (100) and the second coupling element (102) positively intermesh when the reference device (50) assumes the first and/or the at least one second reference position.

26. Reference device according to Claim 24 or 25, wherein the first coupling element (100) is arranged on the reference element base unit (52) and the second coupling element (102) is arranged on the first frame element (60).

27. Reference device according to one of Claims 24 to 26, wherein the first coupling element (100) is movable relative to the second coupling element (102) in a coupling direction (118) for movement of the first and the second coupling elements (100, 102) from a decoupling position, in which the first and the second coupling elements (100, 102) are separated from one another, into a coupling position, in which the reference device (30) assumes the first and/or the at least one second reference position.

28. Reference device according to Claim 27, wherein the coupling direction (118) runs parallel to the base plane (94) and/or parallel to the reference element plane (58).

29. Reference device according to one of Claims 24 to 28, wherein the first coupling element (100) comprises a recess (108) and the second coupling element (102) comprises a projection (114) that can be inserted into the recess (108).

30. Reference device according to one of Claims 24 to 29, wherein a locking element (112) is provided for locking the first and the second coupling elements (100, 102) in the coupling position.

31. Reference device according to Claims 29 and 30, wherein the projection is provided with a thread and the locking element (112) is a nut with an internal thread corresponding to the thread.

## Revendications

1. Dispositif de référenciation de la rotule (30) destiné à déterminer la position dans l'espace d'une rotule (32) d'une articulation du genou (44) de l'homme lors d'une intervention chirurgicale assistée par navigation, comportant un corps de base (48), apte à être fixé à la rotule (32), un élément de référence (50) apte à être détecté par un dispositif de détection (14), **caractérisé en ce que** le dispositif de référenciation de la rotule (30) comporte un dispositif d'assemblage (100, 102) pour l'assemblage amovible du corps de base (48) et de l'élément de référence (50) dans une première position de référence et uniquement dans une seule autre position de référence, à savoir une deuxième position de référence différente de la première position de référence et tournée de 180° par rapport à la première position de référence.

2. Dispositif de référenciation selon la revendication 1, dans lequel le corps de base (48) comporte un premier élément de cadre (60) et au moins un deuxième élément de cadre (74), sachant que dans une position désolidarisée, ledit au moins un deuxième élément de cadre (74) peut être déplacé par rapport au premier élément de cadre (60) et, dans une position fixée, le première élément de cadre (60) et ledit au moins un deuxième élément de cadre (74) sont fixés l'un à l'autre de manière immobile.

3. Dispositif de référenciation selon la revendication 2, dans lequel le premier élément de cadre (60) et/ou ledit au moins un deuxième élément de cadre (74) comportent un guidage (70, 72) pour respectivement l'autre élément de cadre (60, 74), destiné à guider un mouvement relatif du premier et dudit au moins un deuxième élément de cadre (60, 74) dans la position désolidarisée.

4. Dispositif de référenciation selon la revendication 2 ou 3, dans lequel le premier élément de cadre (60) peut être déplacé en translation par rapport audit au moins un deuxième élément de cadre (74).

5. Dispositif de référenciation selon la revendication 3 ou 4, dans lequel le guidage (70, 72) définit une voie courbe pour un mouvement relatif du premier et dudit au moins un deuxième élément de cadre (60, 74) dans la position désolidarisée.

6. Dispositif de référenciation selon l'une quelconque des revendications 2 à 5, dans lequel il est prévu un dispositif de blocage (80) pour l'assemblage amovible du premier élément de cadre (60) avec ledit au moins un deuxième élément de cadre (74) dans la position fixée.

7. Dispositif de référenciation selon la revendication 6, dans lequel le dispositif de blocage comporte un élément d'actionnement (80) pour amener le dispositif de blocage de la position désolidarisée dans la position fixée et inversement.

8. Dispositif de référenciation selon la revendication 7, dans lequel le dispositif de blocage (80), dans une position de base, occupe la position fixée.

9. Dispositif de référenciation selon la revendication 8, dans lequel le dispositif de blocage comporte un élément précontraint pour amener automatiquement l'élément d'actionnement (80) dans la position de base.

10. Dispositif de référenciation selon l'une quelconque des revendications précédentes, dans lequel au moins trois points d'appui (88, 92) sont prévus sur le corps de base (48) et sont destinés à venir en appui sur la rotule (32), lesdits au moins trois points d'appui (88, 92) définissant un plan de base (94).

11. Dispositif de référenciation selon la revendication 10, dans lequel au moins un des points d'appui (88, 92) est défini par une pointe (88, 92) d'un goujon (86, 90).

12. Dispositif de référenciation selon la revendication 11, dans lequel tous les points d'appui (88, 92) sont définis par une pointe (88, 92) d'un goujon (86, 90).

13. Dispositif de référenciation selon la revendication 11 ou 12, dans lequel le premier élément de cadre (60) et ledit au moins un deuxième élément de cadre (74) portent chacun au moins un goujon (86, 90).

14. Dispositif de référenciation selon l'une quelconque des revendications 11 à 13, dans lequel au moins un goujon (86, 90) est disposé de manière réglable par rapport à un élément de cadre (60, 74).

15. Dispositif de référenciation selon l'une quelconque des revendications 10 à 14, dans lequel les points d'appui (88, 92) sont dirigés sensiblement l'un vers l'autre.

16. Dispositif de référenciation selon l'une quelconque des revendications précédentes, dans lequel l'élément de référence (50) porte au moins trois éléments d'émission (42) aptes à être détectés par le dispositif d'identification (14).

17. Dispositif de référenciation selon la revendication 16, dans lequel lesdits au moins trois éléments d'émission (42) sont des éléments d'émission passifs (42).

18. Dispositif de référenciation selon la revendication 16 ou 17, dans lequel les éléments d'émission sont des billes (42) réfléchissant un rayonnement électromagnétique.

19. Dispositif de référenciation selon l'une quelconque des revendications 16 à 18, dans lequel lesdits au moins trois éléments d'émission (42) définissent un plan (58) de l'élément de référence.

20. Dispositif de référenciation selon la revendication 19, dans lequel le plan (58) de l'élément de référence, dans la première position de référence, est orienté parallèlement au plan (58) de l'élément de référence dans ladite au moins une deuxième position de référence.

21. Dispositif de référenciation selon l'une quelconque des revendications 16 à 20, dans lequel l'élément de référence (50) comporte un corps de base (52) et lesdits au moins trois éléments d'émission (42) sont maintenus sur le corps de base (52) de l'élément de référence.

22. Dispositif de référenciation selon la revendication 21, dans lequel lesdits au moins trois éléments d'émission (42) sont maintenus de manière amovible sur le corps de base (52) de l'élément de référence.

23. Dispositif de référenciation selon la revendication 21 ou 22, dans lequel le corps de base de l'élément de référence possède la forme d'une bague (52) plane, plate.

24. Dispositif de référenciation selon l'une quelconque des revendications précédentes, dans lequel le dispositif d'assemblage (100, 102) comporte un premier élément de couplage (100) et un deuxième élément de couplage (102), le premier élément de couplage (100) étant disposé sur l'élément de référence (50) et le deuxième élément de couplage (102) étant disposé sur le corps de base (48).

25. Dispositif de référenciation selon la revendication 24, dans lequel le premier élément de couplage (100) et le deuxième élément de couplage (102) s'engagent l'un dans l'autre par conjugaison de forme lorsque le dispositif de référence (50) est amené dans la première et/ou ladite au moins une deuxième position de référence.

26. Dispositif de référenciation selon la revendication 24 ou 25, dans lequel le premier élément de couplage (100) est disposé sur le corps de base (52) de l'élément de référence et le deuxième élément de couplage (102) est disposé sur le premier élément de cadre (60).

27. Dispositif de référenciation selon l'une quelconque des revendications 24 à 26, dans lequel le premier élément de couplage (100) peut être déplacé par rapport au deuxième élément de couplage (102) dans une direction de couplage (118) pour amener le premier et le deuxième élément de couplage (100, 102) depuis une position de découplage, dans laquelle le premier et le deuxième élément de couplage (100, 102) sont séparés l'un de l'autre, dans une position de couplage, dans laquelle le dispositif de référenciation (30) occupe la première et/ou ladite au moins une deuxième position de référence.

28. Dispositif de référenciation selon la revendication 27, dans lequel la direction de couplage (118) est parallèle au plan de base (94) et/ou parallèle au plan (58) de l'élément de référence.

29. Dispositif de référenciation selon l'une quelconque des revendications 24 à 28, dans lequel le premier élément de couplage (100) comporte un évidement (108) et le deuxième élément de couplage (102) comporte une saillie (114) propre à être introduite dans l'évidement (108).

30. Dispositif de référenciation selon l'une quelconque des revendications 24 à 29, dans lequel il est prévu un élément de verrouillage (112) destiné à verrouiller le premier et le deuxième élément de couplage (100, 102) dans la position de couplage.

31. Dispositif de référenciation selon les revendications 29 et 30, dans lequel la saillie comporte un filetage et l'élément de verrouillage (112) est un écrou avec un taraudage correspondant au filetage.
